# EUROPEAN PATENT APPLICATION

(11) **EP 3 741 323 A1**
(43) Date of publication of application: **25.11.2020**
(21) Application number: 20175758.0
(22) Date of filing: 20.05.2020
(51) Int. Cl.: A61C 7/00, A61C 9/00, A61C 19/045, A61B 90/00, A61B 34/10

(54) **METHOD FOR PROVIDING AN ANATOMICAL PROTOTYPE**

(30) Priority: 22.05.2019 IT 201900007087
(71) Applicant: Just Digital S.r.l., 40134 Bologna (IT)
(72) Inventor: VITALI, Tommaso, 06061 CASTIGLIONE DEL LAGO PG (IT)
(74) Representative: Modiano, Micaela Nadia

(57) **Abstract**

A method for providing an anatomical prototype (100), adapted to simulate the outcome of a treatment that can be performed on a subject (2), which consists in:
a. acquiring at least one digital image, illustrating at least one portion of the oral cavity (3) of the subject (2);
b. obtaining an at least partial digital three-dimensional reproduction of the oral cavity (3) of the subject (2), in particular in order to acquire information on the dental arches, on dental occlusion and/or on the anatomy of the arches;
c. providing the digital image and the digital three-dimensional reproduction to at least one software application, adapted to be run on an electronic processing device associated with a respective display and provided with instructions for the editing of two-dimensional and three-dimensional figures;
d. calibrating the software application on the basis of a known length reference, which can be acquired in the digital image and/or in the digital three-dimensional reproduction;
e. by means of the software application, superimposing the digital image on the three-dimensional reproduction in order to view a first three-dimensional model which illustrates at least partially the current mouth of the subject (2);
f. modifying the first three-dimensional model by means of the software application, until a second three-dimensional model is generated which illustrates at least partially a digital representation of a target denture (7), which can be obtained on the subject (2) as an outcome of the treatment to be performed in the oral cavity (3) of the subject (2);
g. providing a three-dimensional prototype (100) of the second three-dimensional model.

## Description

The present invention relates to a method for providing an anatomical prototype.

In the field of dentistry, often there is the need to plan and define for a patient a path which, for therapeutic, aesthetic or other purposes, has the goal of replacing, creating or reconstructing teeth and tissues that in any case are related to the oral cavity.

This goal, for example, can be sought as a consequence of pathologies or traumas of various kinds, which have caused the loss or deterioration of one or more teeth (or of entire dental arches, in the case of completely edentulous patients).

Therefore, these circumstances may require the intervention of the surgeon or odontologist, to indeed give back to the patient the correct functionality of the oral cavity, or more simply can cause an unpleasant aesthetic appearance, which in any case the patient wishes to remedy.

Moreover, the legitimate desire to improve one's own aesthetic appearance and one's own smile in particular may also arise in healthy subjects if they struggle to live with some characteristics of their face.

In all these contexts, before acting directly in the mouth of the patient, it is preferable or necessary to provide a three-dimensional prototype that simulates the mouth itself (or a part thereof), with the appearance that one presumes or wishes it to assume at the end of the hypothesized path.

This prototype of the "future" mouth (denture) has first of all a predictive function. By simulating the procedure, in fact, it allows the odontologist and the patient to assess its outcome and therefore its appreciation. Moreover, it can constitute a useful reference onto which to model and build a prosthesis (for example for completely or partially edentulous patients).

Whatever the purpose for which the prototypes are intended (among the ones mentioned above or others still), known methods for providing them are not free from drawbacks.

They in fact entail frequent sessions which the patient must undergo in order to be able to obtain the impressions that are necessary to provide models of the oral cavity of the patient. These models can be then processed and shaped indeed in order to try to simulate the outcome that one wishes to obtain with the hypothesized path.

This is evidently a rather laborious procedure which forces a considerable waste of time, both for the patient, who has to go to the outpatient center multiple times, and for the odontologist, who has to provide the model manually and in multiple steps to then modify it appropriately.

Moreover, the actual match between the outcome of the procedures and the prototype provided beforehand depends on the actual manual skill of the person who provides the prototype itself, thereby determining a poor reproducibility of the process and, not infrequently, surprises for the patient when the result is different from expectations.

For these reasons also, and especially when the prototype is not strictly necessary, sometimes it is preferred to avoid its provision, thereby increasing the risk that the result is far from the expectations of the patient.

The aim of the present invention is to solve the problems described above, by devising a method that allows to provide an anatomical prototype in a practical and easy manner.

Within this aim, an object of the invention is to devise a method that provides an anatomical prototype with effective predictive capabilities, i.e., capable of anticipating faithfully the outcome of procedures and treatments of various kinds on the oral cavity of a subject.

Another object of the invention is to devise a method that allows to provide an anatomical prototype without requiring the subject who is receiving the treatments to undergo frequent and prolonged sessions.

Another object of the invention is to provide a method that ensures high reliability in operation and allows to provide an anatomical prototype without requiring laborious and complex manual working activities for the preparation of impressions and models.

Another object of the invention is to devise a method that provides an activity sequence that is alternative to those of methods of the known type.

Another object of the invention is to provide a method that can be performed easily starting from commonly commercially available elements and materials.

Another object of the invention is to provide a method that can be performed simply and at low costs.

This aim and these and other objects which will become better apparent hereinafter are achieved by a method according to claim 1.

Further characteristics and advantages of the invention will become better apparent from the description of a preferred but not exclusive embodiment of the method according to the invention, illustrated by way of non-limiting example in the accompanying drawings, wherein:

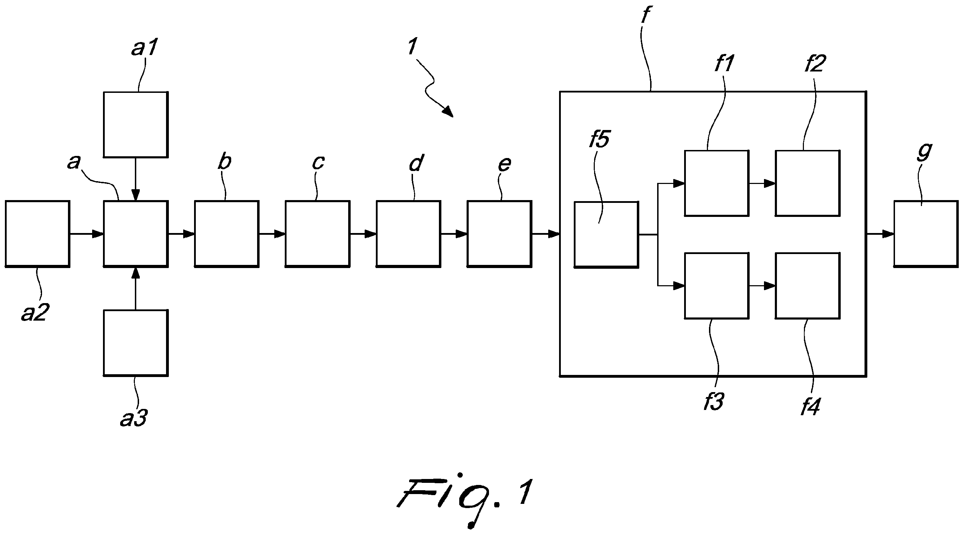

Figure 1 is a block diagram of the method according to the invention;
Figures 2 and 3 are views of two examples of what can be acquired with the execution of step a. of the method according to the invention;
Figure 4 is a front elevation view of a three-dimensional prototype that can be provided by means of the method according to the invention.

With reference to the figures, the reference numeral 1 generally designates a method for providing an anatomical prototype 100.

The prototype 100 is adapted to simulate (or anticipate) the outcome of a treatment that can be performed on a subject 2. Said subject, before the treatment and the execution of the method 1 according to the invention, can have all or some teeth missing or still have them all.

In greater detail, as will become better apparent in the pages that follow, the prototype 100 that is obtained by means of the method 1 is a three-dimensional object that anticipates the shape that will be assumed in the subject 2 by the teeth and by the dental arches (or by at least a part thereof), indeed at the end of the treatment that one must perform or wishes to perform, for various purposes (therapeutic and/or aesthetic, or others).

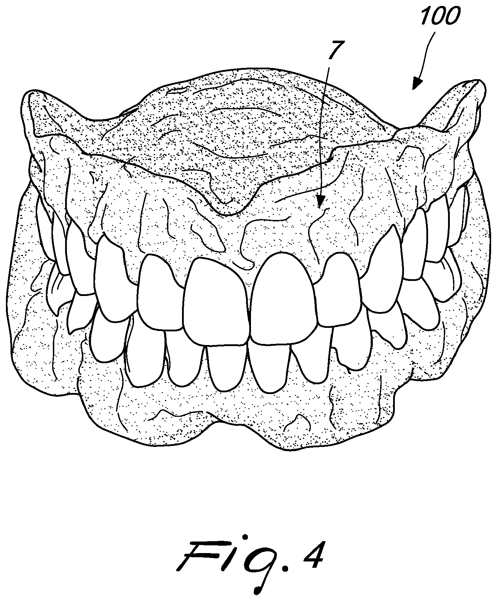

In this regard, Figure 4 shows, merely by way of non-limiting example of the application of the invention, a prototype 100 of the prosthetic type, which simulates the outcome of a treatment intended for an edentulous subject 2.

In fact, in some cases the prototype 100 can simulate the shape of prosthetic structures that will have to be installed in the mouth of the patient as a replacement of missing teeth; as an alternative, if the subject 2 still has his own teeth (or existing prosthetic structures), the prototype 100 can simulate the outcome of treatments or therapies of various kinds intended to modify their structure and shape, as well as those of the surrounding tissues. Furthermore, the prototype 100 can be a combination of the two preceding cases.

The subject 2 for whom the treatment is intended may have undergone traumas and be affected by pathologies of any kind, and in this case the method 1 can have the function of anticipating the outcome of actions (surgical or not) aimed at restoring the original denture and/or an optimum smile, or in any case at least improve them, giving a more pleasant appearance to the subject 2.

At the same time, the subject 2 for whom the treatment is intended may also be healthy in every respect but dissatisfied with his current appearance, and in this case the treatment is therefore of the type of an aesthetic procedure.

The prototype 100 can also be used for other purposes, for example it can be used by the surgeon as a base onto which to check or model further prostheses or other devices (to be installed in the mouth of the subject 2, after the hypothesized treatments). Additional purposes and goals, which might lead to the execution of the method 1 and to the provision of the prototype 100 and are in any case to be understood as included within the protective scope claimed herein, are not excluded in any case.

In any case, it is specified furthermore that the surgeon, even before starting the method 1, may already have in mind the actions to be performed and their effect on the teeth, and in this case the method 1 is aimed at providing a prototype 100 that simulates the outcome of these already-known actions, in order to share it with the subject 2. In other cases it is indeed the method 1 that allows the surgeon to identify the actions that are most suitable for the preset goal, and therefore to model a prototype 100 that complies with these actions and with the treatment that indeed by means of the method 1 has been identified as the best for the requirements of the subject 2.

According to the invention, the method 1 consists, in a step a., in acquiring at least one digital image (of the 2D or 3D type), which shows at least one portion of the oral cavity 3 of the subject 2. It is specified in this regard that the digital image can show the at least partial current denture of the subject 2 and therefore all the teeth or only some of them (be they natural or prosthetic), as a function of the specific requirements.

As already noted, the method 1 can be performed also on (fully or partially) edentulous patients and therefore the oral cavity 3 visualized with the digital image lacks teeth. In this case, therefore, the possibility is provided to acquire the digital image (in step a., indeed) after applying at least one occlusal rim, for example made of wax and/or resin, which indeed simulates the space occupation of a dental arch. The rim, or a part thereof, is therefore shown by the digital image.

In particular, in an embodiment of considerable practical interest which in any case does not limit the application of the invention, step a. entails acquiring the digital image by means of an electronic device chosen from a still camera (digital or not, in this last case subsequently providing digitization), a face scanner, a smartphone, a tablet.

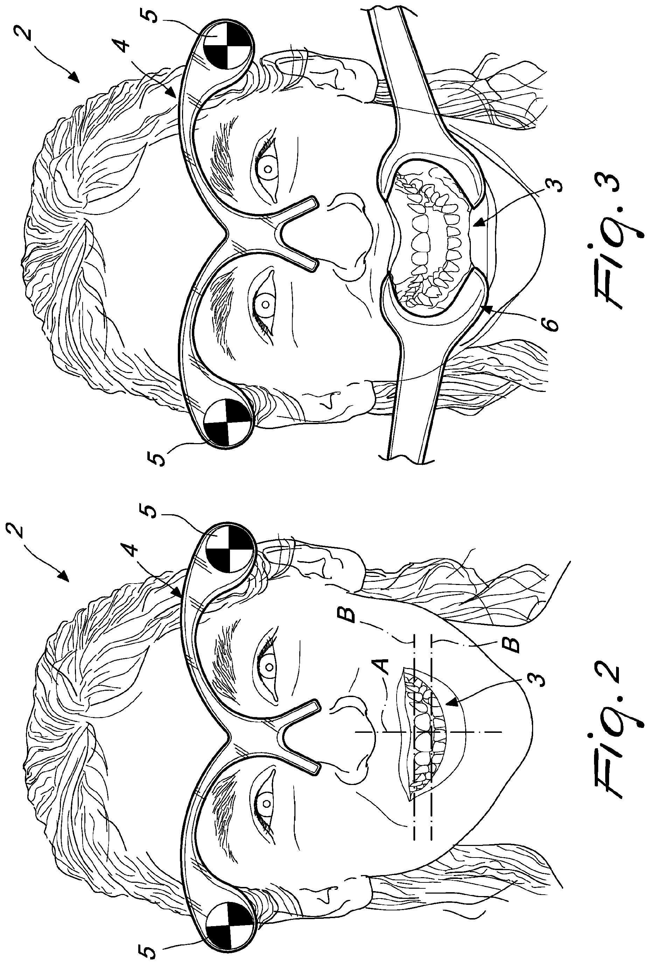

It is stressed that the digital image can be obtained by simply asking the subject 2 to smile (Figure 2), so as to expose part of the current denture (and/or of the rim), but allowing in any case to appreciate the shape of the incisal plane with respect to the lower lip, as well as the breadth of the lateral corridors.

As an alternative, the method 1 according to the invention entails, in a substep a1. that precedes step a., installing a retractor 6 in the mouth of the subject 2 (Figure 3). By virtue of the retractor 6, the digital image substantially shows the entire dental arches of the subject 2 (or the rim), even for the portions that are normally hidden by the lips or cheeks. In greater detail, the digital image is preferably provided while the arches are partially open and allows to assess correctly both the parallel arrangement between the bipupillary and occlusal planes and the match between the upper and/or lower central line A and the inter-incisor line.

Moreover, it is specified that the method 1 according to the invention entails the acquisition of two or more digital images (for example, one in which the retractor 6 is installed on the subject 2 and the other one in which the latter is absent), to be both used in the subsequent steps or among which to choose the most appropriate one only during the subsequent steps.

After step a., the method 1 according to the invention entails, in a step b., obtaining an at least partial digital three-dimensional reproduction of the oral cavity 3 of the subject 2, in particular to acquire information on the dental arches (on a part thereof or in their entirety), on dental occlusion and/or on the anatomy of the arches. More generically, step b. entails acquiring information on the three-dimensional structure of the oral cavity of the subject 2.

As is known, the expression "dental occlusion" identifies the mutual relationships that the mastication surfaces of the teeth assume in the act of closing the dental arches. Step b., performed for example by picturing from above the lower arch and the upper arch from below, allows to obtain important information on the mutual arrangement of these arches, on their curvature and in general on their three-dimensional extension and their shape. This information returns a "three-dimensional" picture of the mouth of the subject 2 and therefore in a way is complementary to the information that can be deduced from the digital image acquired in step a., which obviously shows in a more limited manner the front appearance of said arches.

In particular, in a preferred embodiment, step b. entails obtaining the three-dimensional reproduction by means of a scanner, such as for example an intraoral scanner which is already commercially available or in any case of a commercial type, such as the one marketed with the commercial name "Trios" by the Danish company 3 Shape (mentioned here merely by way of example).

As an alternative, step b. entails obtaining the three-dimensional reproduction starting from impressions of the arches (subsequently digitized), provided according to known methods.

In a step c., the method 1 according to the invention entails providing the digital image and the digital three-dimensional reproduction to at least one software application, adapted to be run on an electronic processing device associated with a respective display (a monitor, a screen, chosen of any type). This software application is provided with instructions for the editing of two-dimensional and three-dimensional figures. The software application is in any case capable of operating in a three-dimensional environment.

The electronic device is typically a personal computer, but it can also be a server computer, a mobile device such as for example a tablet, or others, without thereby abandoning the protective scope claimed herein. The application may be provided ad hoc or may be a sort of evolution of an already commercially available application (for example the DSS software, sold by the Italian company Digital Smile System), to be integrated indeed with the necessary functions. As an alternative, the application may more simply be chosen among those commercially available, already capable of operating in a 3D environment and of performing the functions that will be described in the paragraphs that follow (for example the Exocad software marketed by the company Exocad GmbH).

Subsequently, the method 1 according to the invention entails, in a step d., calibrating the software application on the basis of a known length reference, which can be acquired in the digital image and/or in the digital three-dimensional reproduction (which in the meantime will have been shown on the display).

The reference can be visible on the face of the subject 2 as well as in its vicinity (it can be applied to a structure arranged to the side of the face, for example) and can be of any type without abandoning the scope of application claimed herein. In greater detail, reference systems or means of any kind, so long as they indeed have at least one known length reference, are arranged on the face of the patient.

In particular, in an embodiment of the invention that is of considerable practical interest, the method 1 entails, in a step a2. that precedes step a., applying to the face of the subject 2 a pair of goggles 4 (even without lenses) which have a pair of markers 5 arranged at a known distance. In this case, therefore, it is indeed the center distance between these markers 5 that constitutes the known length reference to be pictured and acquired with the digital image in step a. As can be also seen from the accompanying figures, the markers 5 are arranged (preferably but not exclusively) at the ends of the front portion of the frame of the pair of glasses 4.

As an alternative to or as a complement to step a1., the method 1 entails, in a step a3. that precedes step a., measuring the length of an anatomical element of the face (teeth, nose, mouth, pupil, etc.) of the subject 2, with said anatomical element indeed constituting the known length reference to be pictured and acquired with the digital image in step a..

Whatever the reference actually chosen (and which may also be acquirable in the digital three-dimensional reproduction, as already noted), in order to be able to process the digital image correctly by means of the application (in the manners described hereinafter), being able to rely on a precise match between the dimensions and proportions between pixels on the screen and the actual ones of the subject 2, step d. clearly has a crucial role, since it allows to know with precision the respective "scale factor".

It is in fact sufficient to provide the application with the information related to the measurement in centimeters (or other unit of measurement deemed appropriate) of the known length (whether related to the center distance between the markers 5, chosen for example equal to 17 cm, or other), in order to allow said application to know and return the measurements in centimeters (or other unit of measurement) of any element in the image, linking the measurements in pixels on the latter to the measurement in pixels of the distance between the references 4, of which the measurement also in centimeters (or other unit of measurement) is indeed known.

The method 1 according to the invention then entails, in a step e. to be performed by means of the software application, superimposing the digital image on the three-dimensional reproduction. This step e. is performed taking care to align the portion of the oral cavity 3 that has been acquired (and therefore the portion of the current denture and/or of the wax rim) with the part of the arches obtained (or in any case with the structures that can be acquired in the three-dimensional reproduction).

It should be noted that step e. (as besides step d.) can be performed by the software application by working in a 3D (three-dimensional) environment: applications capable of running it (including the ones already mentioned) are in fact already commercially available; as an alternative, the software application provided ad hoc shall have to provide functions capable of performing said step e. automatically or with the intervention of the user.

In any case, by virtue of the superimposition thus performed, it is possible to view (on the display) a first three-dimensional model which illustrates at least partially the current mouth of the subject 2 (the teeth and the arches of the subject 2 and/or the wax rim are at least partially visible on the first model). It is stressed that the model may show the totality of the arches and teeth or only a part thereof (or even just the rim), as a function of the specific requirements and of what has been previously acquired and obtained in steps a. and b..

The method 1 according to the invention then entails, in a step f., modifying the first three-dimensional model (and in particular the digital image) by means of the software application (by resorting to functions that are already available or are provided specifically), until a second three-dimensional model is generated which illustrates at least partially a digital representation of a target denture 7, which can be obtained on the subject 2 as an outcome of the treatment to be performed in the oral cavity 3 of the subject 2 (as a replacement of the current denture or to provide a prosthetic structure to an edentulous patient). Step f. also can be performed by working in a 3D environment and by resorting to the software applications already mentioned or to others that are commercially available, or to a specifically created application.

It is appropriate to stress that in the execution of step f. (and of the method 1 in general) it is possible to consider some constraints dictated by the specific requirements of the subject 2, for example if he requires specific procedures or therapies on one or more deteriorated or missing teeth (or if he wishes a certain type of aesthetic procedure). In this case, the actions and modifications to be applied to the teeth may be already known and the prototype 100 to be obtained must comply with these constraints: in step f. the surgeon (or the person who performs the method 1) shall have to modify the first three-dimensional model so as to simulate and anticipate the outcome of the interventions and of the therapies already decided and the treatment to be performed shall have to be aimed at meeting these requirements.

As an alternative, the various actions to be performed might not be known and the surgeon (typically but not exclusively an odontologist) shall simply have to consider the reason that led the subject 2 to request the treatment. In this case, in step f. the surgeon may test (by simulating them) various actions, until the target denture 7 that best fits the face of the subject 2 and its shape (indeed in view of his requirements) is identified and created. The treatment to be performed physically on the face of the subject 2 is therefore identified indeed by means of the execution of the method 1 and is the one capable of providing the modifications made in step f., which are indeed required in order to obtain the target denture 7 identified at the end of this last step.

It is stressed that the target denture 7 may simulate a prosthetic structure to be installed in the oral cavity 3 of the subject 2 or may anticipate the outcome of treatments to be performed on the hard and soft tissues of the face and/or oral cavity.

It should be noted that the three-dimensional model can already be shared with the subject 2 (to test its approval and/or obtain his consent to proceed with the subsequent steps), or in any case analyzed by the surgeon to assess its appreciation and therefore decide whether the target denture 7 that has been identified actually meets the requirements and taste of the subject 2.

After obtaining the second three-dimensional model (and optionally after sharing it with the subject 2), the method 1 according to the invention entails, in a step g., providing a three-dimensional prototype 100 of the second three-dimensional model.

In particular, in one embodiment of considerable practical interest, step g. entails generating a file of the STL type on the basis of the second three-dimensional model. This generation can be performed by dedicated software, even obtained commercially: for example, a suitable software might be the one known as DSS CAD, marketed by the Italian company EGS Srl. Then step g. entails modeling the actual prototype 100, providing the STL file to software and 3D printers (or also to milling machines) that adopt 3D SLA technologies (for example Form 2, of the United States company Formlabs Inc.) to provide prototypes 100 made of photopolymer resin or of another material. Examples of this resin are known in the field with the acronyms GPWH02 or GPGR03 (but it is stressed that other materials also are expected to be used to provide the prototype 100, as a function of the specific requirements, without thereby abandoning the protective scope claimed herein).

It should be noted that the methods for creating the three-dimensional model (step f.) may be any, as a function of the requirements and of the possibilities offered by the software application chosen to perform the method 1 according to the invention. In any case, some practical examples of creation are provided hereinafter which constitute a corresponding number of preferred embodiments, which in any case do not limit the invention and can be adopted for a subject 2 who has, in the portion of the oral cavity 3 that was acquired in step a., an at least partial current denture (and therefore for a subject 2 who is not completely edentulous).

In particular, therefore, step f. entails, in a substep f1., replacing the current denture with a corresponding portion of a sample denture, chosen from a library associated with the software application.

The library contains a range of possible sample dentures saved previously on a memory unit associated with the application and/or with the electronic device. These sample dentures might be saved in the library only in their two-dimensional representation or might be associated with a respective three-dimensional representation.

Substep f1. can be performed automatically by the application, since provisions are made for providing it with instructions for choosing the sample denture that is most suited to the specific circumstances, on the basis of shapes (of the teeth) that belong to the oval, triangular or square type. As an alternative, substep f1. can be performed directly by the surgeon (odontologist or other), indeed by choosing from the library the one that he deems best suited to the face of the subject 2, to his psychological profile, or to his aesthetic requests.

After substep f1., the method 1 entails, in a substep f2., altering the dimensions and proportions of the teeth, until the second three-dimensional model is generated.

In substep f2., then, the surgeon (or someone on his behalf) can modify the chosen sample denture if he deems further adaptations or modifications to be necessary in order to achieve the target denture 7. In any case, the possibility to maintain, for the second three-dimensional model, the actual chosen sample denture, as it was stored in the respective library, if it is deemed already satisfactory, is not excluded.

It should be noted that the application allows the surgeon or in any case the operator to position on the digital image two horizontal lines B, which are aligned with the upper and lower margins of the central incisors (of the current denture, in the upper and/or lower arch). The center distance between the two horizontal lines B (which are shown for greater clarity in Figure 2) thus corresponds to the center distance between the incisal border and the cervical border between which the incisors of the target denture 7 are to be positioned, thus providing a visual indication of their maximum allowable size (on which moreover the dimensions of the other teeth also are to be modeled proportionally).

As an alternative, in a second embodiment of the method 1 according to the invention, which is in any case intended for a subject 2 who has an at least partial current denture in the portion of the oral cavity 3, step f. entails, in a substep f3., repositioning the latter inside the oral cavity 3.

In this case, therefore, the method 1 entails (re)using indeed the current denture of the subject 2 to create the second three-dimensional model and the target denture 7, modifying the position of the existing teeth inside the mouth. This is a solution that may produce higher approval in the subject 2, since the simple repositioning of the current denture, instead of the complete replacement provided in the preceding case, attenuates the surprise effect and the feeling of extraneousness that otherwise the subject 2 might feel upon looking at himself in the mirror at the end of the treatment.

It should be noted that the current denture, used in substep f3., might in turn be added to the library or stored separately, as a sort of customized individual library, even for future uses.

The repositioning performed in step f3. might already be sufficient to determine the second three-dimensional model, or step f. might provide, in a substep f4. that follows the step f3., for altering the dimensions and proportions of the teeth, until the second three-dimensional model is generated (in a manner that is similar to what has been observed for substep f2., the same remarks made in that case being therefore again valid).

Usefully, step f. might provide, in a substep f5. that precedes substep f1. and/or substep f3., for performing a facial analysis of the digital image, in order to identify at least one parameter that describes the symmetry and proportions of the face and/or the current denture. This allows to perform in an optimum manner substep f1. (especially) and/or substep f3. since, having acquired this parameter, the application can easily position the sample denture and/or reposition the current denture in the mouth of the subject 2.

In particular, the parameter to be identified in substep f5. is chosen among the superior and/or inferior median line A (which may coincide or not), the bipupillary line, the occlusal plane, the longitudinal lines of each tooth, the height to width ratio of each tooth, the ratio between the dimensions of the various teeth, the radius of curvature of the gum parables, the radius of curvature of the lower profile of the upper dental arch and/or of the upper profile of the lower dental arch.

Even more particularly, in the preferred embodiment of the invention step f5. entails at least identifying the median line A (shown for the sake of greater clarity in Figure 2).

The execution of the method 1 and the ways in which the prototype 100 is provided have therefore already been described in detail in the preceding pages; in any case, a brief summary thereof is provided hereafter.

If it is necessary to set up a treatment on the face of a subject 2 (a treatment which can be constituted by the provision of a prosthetic structure), the method 1 according to the invention allows to predict its outcome, anticipating the effect of the various actions to be performed, be they already known or not (in this last case, allowing the surgeon to identify the most suitable ones indeed by means of the simulation of their effects).

In order to be able to obtain this particular result, the subject 2 must undergo a single session, in which the surgeon (or someone on his behalf) can acquire a digital image that illustrates at least one portion of the oral cavity 3 of the subject 2. In the same session (or even in another one, if necessary), it is necessary to obtain a digital three-dimensional reproduction of the dental arches of the subject 2. The digital image and the three-dimensional reproduction are then supplied to a software application which, calibrated appropriately, is capable of superimposing them and therefore allows first of all to view a first three-dimensional model that illustrates at least partially the current mouth of the subject 2. On this first model the surgeon can intervene (by utilizing the editing tools of the software application) until a second three-dimensional model is generated which illustrates at least partially the digital representation of the target denture 7. Finally, the method 1 provides a three-dimensional prototype 100 of the second three-dimensional model.

It has thus been shown that the method 1 according to the invention allows to obtain an anatomical prototype 100, to be shown to a subject 2 before beginning the treatment that one wishes to perform in the mouth of the latter. The prototype 100 in fact replicates the target denture 7, i.e., what the surgeon (odontologist or other) seeks to obtain with the treatment, and therefore allows to share it in advance with the subject 2. Such subject can thus confirm his approval, avoiding the risk of unpleasant surprises, which might occur if he should find, once the treatment has been performed, that his mouth has been modified in a manner that does not comply with his expectations.

More generally, the anatomical prototype 100, obtained with the method 1, performs a fundamental predictive role, simulating and anticipating faithfully the outcome of procedures and treatments of various kinds on the oral cavity 3 of the subject 2.

The prototype 100, by replicating effectively the target denture 7, and therefore the configuration assumed after the treatment by the mouth of the subject 2, also allows the surgeon to verify compatibility with any prostheses or implants to be installed subsequently, thus avoiding once again the identification of a criticality only after the mouth of the subject 2 has been altered.

These unquestionably important results are obtained by means of a method 1 which can be performed in a fully practical and easy manner, requiring a small number of simple activities that can be performed mostly in front of the screen of a personal computer and requiring a limited participation of the subject 2.

In greater detail, it should be noted that the method 1 provides only two steps (a. and b.) which are implemented in an outpatient center with the participation of the subject 2. Therefore, the latter can undergo a single session (during which steps a. and b. can be completed rapidly) and then waits to receive the prototype 100 directly. Differently from what occurs according to the background art, the method 1 therefore allows to obtain a prototype 100 without requiring the subject 2 to undergo frequent and prolonged sessions, thus causing him a very modest annoyance. Moreover, the only session requires taking one or more photographs (or obtaining the digital image or images in another manner) and subjecting the subject 2 to optical scanning (or obtaining in another manner the three-dimensional reproduction of the dental arches): these activities are very quick and are certainly quicker than those required to obtain impressions of the dental arches, as indeed provided with the background art.

The subsequent steps c. to f. are instead performed by the surgeon (or by someone on his behalf) by using the software application (working in a 3D - three-dimensional environment), and therefore digitally, being able to remain comfortably seated in front of the display of the electronic device. Finally, step g. entails resorting to a 3D printer, to a milling machine or to other three-dimensional modeling devices. In any case, the method 1 according to the invention spares the surgeon and/or his collaborators laborious and complex manual working activities for the preparation of impressions and models and in general requires a minimum effort thereof.

Moreover, since it is possible to work by using a software application (or a software application package), the method 1 significantly reduces the unpredictability of the outcome, which is instead very present if one resorts to manual activities of the known type.

The invention thus conceived is susceptible of numerous modifications and variations, all of which are within the scope of the inventive concept; all the details may furthermore be replaced with other technically equivalent elements.

In the exemplary embodiments shown, individual characteristics, given in relation to specific examples, may actually be interchanged with other different characteristics that exist in other exemplary embodiments.

In practice, the materials used, as well as the dimensions, may be any according to the requirements and the state of the art.

The disclosures in Italian Patent Application No. 102019000007087 from which this application claims priority are incorporated herein by reference.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A method for providing an anatomical prototype (100), adapted to simulate the outcome of a treatment that can be performed on a subject (2), which consists in:
a. acquiring at least one digital image, illustrating at least one portion of the oral cavity (3) of the subject (2);
b. obtaining an at least partial digital three-dimensional reproduction of the oral cavity (3) of the subject (2), in particular in order to acquire information on the dental arches, on dental occlusion and/or on the anatomy of the arches;
c. providing the digital image and the digital three-dimensional reproduction to at least one software application, adapted to be run on an electronic processing device associated with a respective display and provided with instructions for the editing of two-dimensional and three-dimensional figures;
d. calibrating the software application on the basis of a known length reference, which can be acquired in the digital image and/or in the digital three-dimensional reproduction;
e. by means of the software application, superimposing the digital image on the three-dimensional reproduction in order to view a first three-dimensional model which illustrates at least partially the current mouth of the subject (2);
f. modifying the first three-dimensional model by means of the software application, until a second three-dimensional model is generated which illustrates at least partially a digital representation of a target denture (7), which can be obtained on the subject (2) as an outcome of the treatment to be performed in the oral cavity (3) of the subject (2);
g. providing a three-dimensional prototype (100) of the second three-dimensional model.

2. The method according to claim 1, **characterized in that** said step a. entails acquiring the digital image by means of an electronic device chosen from a still camera, a face scanner, a smartphone, a tablet.

3. The method according to claim 1 or 2, **characterized in that** it entails, in a step a1. which precedes said step a., installing a retractor (6) in the mouth of the subject (2).

4. The method according to one or more of the preceding claims, **characterized in that** it entails, in a step a2. which precedes said step a., applying to the face of the subject (2) a pair of goggles (4) provided with a pair of markers (5) arranged at a known distance, the center distance between said markers (5) constituting the known length reference.

5. The method according to one or more of the preceding claims, **characterized in that** it entails, in a step a3. which precedes said step a., measuring the length of an anatomical element of the face of the subject (2), which constitutes the known length reference.

6. The method according to one or more of the preceding claims, **characterized in that** said step b. entails obtaining the three-dimensional reproduction by means of a scanner or starting from impressions of the arches.

7. The method according to one or more of the preceding claims, **characterized in that**, for a subject (2) who has, in the portion of the oral cavity (3) acquired in said step a., an at least partial current denture, said step f. entails, in a substep f1., replacing the current denture with a corresponding reference denture, chosen from a library associated with said software application, said step f. providing, in a substep f2. which follows said step f1., for altering the dimensions and proportions of the teeth until the second three-dimensional model is generated.

8. The method according to one or more of the preceding claims, **characterized in that**, for a subject (2) who has, in the portion of the oral cavity (3) acquired in said step a., an at least partial current denture, said step f. entails, in a substep f3., repositioning inside the oral cavity (3) the current denture, said step f. providing, in a substep f4., which follows said step f3., for altering the dimensions and proportions of the teeth until the second three-dimensional model is generated.

9. The method according to claim 7 and/or 8, **characterized in that** said step f. entails, in a substep f5. which precedes said substep f1. and/or said substep f3., performing a facial analysis of the digital image in order to identify at least one parameter that is descriptive of the symmetry and proportions of the face and/or of the current denture and consequently execute in an optimum manner said substep f1. and/or said substep f3.

10. The method according to claim 9, **characterized in that** the parameter to be identified in said substep f5. is chosen between the superior and/or inferior median line (A), the bipupillary line, the occlusal plane, the longitudinal lines of each tooth, the height to width ratio of each tooth, the ratio between the dimensions of the various teeth, the radius of curvature of the gum parables, the radius of curvature of the lower profile of the upper dental arch and/or of the upper profile of the lower dental arch.
